# EUROPEAN PATENT SPECIFICATION

(11) **EP 4 540 575 B1**
(45) Date of publication and mention of the grant of the patent: **09.09.2026**
(21) Application number: 23733373.7
(22) Date of filing: 20.06.2023
(51) Int. Cl.: G01B 7/04, G01B 7/06

(54) **A METHOD AND DEVICE FOR DETERMINING THICKNESS AND SOLID FRACTION OF A PHARMACEUTICAL TABLET USING NON-INVASIVE PROCESS ELECTRICAL TOMOGRAPHY**
VERFAHREN UND VORRICHTUNG ZUR BESTIMMUNG DER DICKE UND DES FESTSTOFFANTEILS EINER PHARMAZEUTISCHEN TABLETTE UNTER VERWENDUNG VON NICHTINVASIVER ELEKTRISCHER PROZESSTOMOGRAPHIE
PROCÉDÉ ET DISPOSITIF DE DÉTERMINATION DE L'ÉPAISSEUR ET DE LA FRACTION SOLIDE D'UN COMPRIMÉ PHARMACEUTIQUE À L'AIDE DE TOMOGRAPHIE ÉLECTRIQUE, PROCESSUS NON INVASIF

(30) Priority: 20.06.2022 PT 2022118054
(43) Date of publication of application: 23.04.2025
(60) Divisional application: 26195723.7
(73) Proprietor: Hovione Scientia Limited, Ringaskiddy, Co. Cork (IE)
(72) Inventor: MONTEIRO, Pedro, 2650-123 AMADORA (PT); DURÃO, Pedro, 7300-120 PORTALEGRE (PT); VALENTE, Pedro, 1500-152 LISBOA (PT); SILVA, Rui, 4750-106 ARCOZELO BCL (PT); CARAMELO, Daniel, 1700-141 LISBOA (PT); JOSUE, Sonia, 2500-922 CALDAS DA RAINHA (PT); SANTOS, Paulo, 3045-043 COIMBRA (PT); CARVALHO, Agostinho, 2400-842 LEIRIA (PT)
(74) Representative: Page White Farrer
(86) International application number: PCT/EP2023/066694
(87) International publication number: WO 2023/247572

(56) References cited:
- WO-A1-2019/057802
- US-A- 4 461 363
- US-B2- 7 956 623

## Description

### TECHNICAL FIELD OF THE INVENTION

The present invention is in the technical field of tablet solid fraction. Specifically, the present invention is related to Process Analytical Technologies, preferably inline, to characterize thickness and solid fraction in solid dosage forms. Moreover, hardness determination is also a part of the invention.

### BACKGROUND OF THE INVENTION

The two main quality attributes of pharmaceutical tablets - mechanical strength and dissolution dynamics - are governed by the porosity/solid fraction of the compressed material. During tableting, along with the internal control of the tablet press (if available), a periodic control of tablets characteristics (weight, hardness, thickness and diameter) is performed using equipment such as multi-testers (either on or offline). However, due to the speed of equipment and destructive aspect of the hardness test, typically only an extremely small part of the batch (around 0.01%) can be determined, which lacks batch representativeness. Owing to its important role in tablet characterization, it is highly desirable to have a PAT tool in place to ensure consistency in product quality, particularly in design space applications. Moreover, in technologies like continuous manufacturing, these properties are frequently used in real time to infer other critical quality attributes, such as dissolution performance. Therefore, the availability of a continuous and fast method to determine these properties is a critical resource to monitor and control these processes and an ideal tool to support Real Time Release applications - the control strategy in place might be designed based on the availability and frequency of the determination of these parameters. Consequently, there have been numerous scientific articles aiming at the measurement of the solid fraction in a fast and non-destructive way, such as Terahertz imaging (Muller et al 2012) or Raman spectroscopy (Peeters et al 2016), but it is not clear whether the limitations in each of these techniques can be surpassed and provide a reliable estimate. An alternative technique, which has a great potential for providing a reliable estimate of solid fraction, is electrical capacitance tomography, which has already been demonstrated to be accurate and reliable in multiphase flows at lower solid fractions. Getting the solid fraction from the capacitance measurement is not direct, as the capacitance responds both to the volume and mass of the pharmaceutical tablet, thus, additional information about the sample is usually needed.

In the context of using process tomography in the characterization of final dosage forms, a survey of the literature identified the following contributions in the field of pharmaceutical processes:
US4461363 patent relates to a method of and apparatus for the capacitive weighing of a bounded or unbounded object as it passes between the plates of a capacitor. Although this apparatus can present several configurations for the purpose of weighing samples it does provide information on solid fraction.

US5135113 and US5240118 patent applications depict machines for weighing and classifying tablets, particularly at a high-speed rate. It is the principal object of these inventions to provide high-speed machines for weighing and classifying tablets by capacitance measurement, but no information on solid fraction is captured.

US10801986 patent depicts a monitoring sensor sleeve for channeling a metered amount of powder and herein subjected to a capacitive mass determination. WO2019057802 and US2020217811 relates to measuring the solid fraction of a pharmaceutical substance. It also presents the challenge of an unknown thickness and solid fraction for the pharmaceutical substance. The documents proposes the use of a separate sensor to measure the thickness of the substance. Using a separated distance capacitance sensor is not a viable option since for its use the material composition and density must be exactly the same across all measurements (which is not true since the density variability across the substances is exactly what is trying to be obtained). The results shown are corrected by the thickness of the different tablets, therefore the thickness and solid fraction measurements proposed in the patent are segregated, with the thickness measured at an earlier stage.

EP2740671B1 describes a device for inspecting tablets with a chute and an angled guide surface along with a sensor integrated into the chute for detecting faulty tablets and a passive piezoelectric bending actuator.

JP2009047687A describes an apparatus with both NIR, for active ingredient inspection, and an array of capacitance sensors to inspect volume. Weight and density can be indirectly inferred from tablet press data and NIR, respectively.

US10565855B2 employs an apparatus to enable automatic monitoring of the state of medicine content of a blister card through a capacitive sensor by tracking tablet location.

US20090314944A1 depicts an apparatus using terahertz radiation non-destructive imaging of chemical and/or structural features of the pharmaceutical tablet containing the active pharmaceutical ingredient (API) and excipients (fillers).

DE2502098 describes the use of capacitance to compare the dielectric values of measured tablets with a standard value - therefore inferring if the tablet weight is acceptable. However, this is only a pass or fail method and does not take into consideration the variations of thickness in the multiple tablets being measured. Further, US 7956623B2 describes a container filling machine comprising a sensing device for detecting the integrity of discrete articles for personal treatment to be packaged in a container.

To summarize, current methods can only have a qualitative measurement of the sample or need to resort to other technologies to have a quantitative solid fraction measurement. As robustness is critical in pharmaceutical industry, relying on multiple devices and technologies for a single parameter measurement is not desirable. Contrasting to prior methods, the present invention provides a quantitative measurement of solid fraction using only capacitance measurements, preferably obtained using a single device, allowing for simpler and more robust equipment.

### SUMMARY OF THE INVENTION

In a first aspect the present invention provides a device for obtaining capacitance measurements for use in determining the relative permittivity and thickness of a pharmaceutical tablet, the device comprising multiple capacitance sensors in one or more sensing sections, the multiple capacitance sensors comprising:
(i) a first capacitance sensor for obtaining a first capacitance measurement, the first capacitance sensor comprising a first pair of conducting elements each conducting element having a flat surface, wherein the flat surfaces of the first pair of conducting elements are arranged in parallel such that capacitance measurements can be obtained from a gap therebetween, wherein the first capacitance measurement is to be obtained when the pharmaceutical tablet is positioned in the gap between the flat surfaces of the first pair of conducting elements in a first orientation with respect to the flat surfaces of the first pair of conducting elements;
(ii) a second capacitance sensor for obtaining a second capacitance measurement, the second capacitance sensor comprising a second pair of conducting elements each having a flat surface, wherein the flat surfaces of the second pair of conducting elements are arranged in parallel such that capacitance measurements can be obtained from a gap therebetween, wherein the second capacitance measurement is to be obtained when the pharmaceutical tablet is positioned in the gap between the flat surfaces of the second pair of conducting elements in a second orientation with respect to the flat surfaces of the second pair of conducting elements;

wherein the arrangement of the parallel flat surfaces of the first pair of conducting elements relative to the parallel flat surfaces of the second pair of conducting elements enables the first orientation to be perpendicular to the second orientation such that in one of the first and second orientations the thickness of the pharmaceutical tablet is parallel to the parallel flat surfaces of the pair of conducting elements and in the other of the first and second orientations the thickness of the pharmaceutical tablet is perpendicular to the parallel flat surfaces of the pair of conducting elements, and
wherein the device comprises a controller, wherein the controller is configured to determine the relative permittivity and thickness of the pharmaceutical tablet using the first and second capacitance measurements.

In one example, the device may comprise one sensing section in which the flat surfaces of the first pair of conducting elements are arranged perpendicularly to the flat surfaces of the second pair of conducting elements. Alternatively, the device may comprise two sensing sections arranged in series, wherein the first pair of conducting elements are arranged in the first sensing section and the second pair of conducting elements are arranged in the second sensing section, wherein the second pair of conducting elements are arranged perpendicularly to the first pair of conducting elements

In a further example, which is described below but which is not within the scope of claim 1, the device may comprise two sensing sections arranged in series, wherein the first pair of conducting elements are arranged in the first sensing section and the second pair of conducting elements are arranged in the second sensing section, and the device comprises a reorientation element to reorientate the pharmaceutical tablet when in the device between the first orientation in the first sensing section and the second orientation in the second sensing section. The reorientation element may, for example, comprises a funnel.

In the device the first pair of conducting elements can be a first pair of parallel plates and the second pair of conducting elements can be a second pair of parallel plates.

In particular, the controller may comprise reference data correlating relative permittivity with solid fraction for a plurality of reference tablets of varying thickness and having the same chemical composition and other dimensions as the pharmaceutical tablet, wherein the controller is configured to determine the solid fraction of the pharmaceutical tablet using the determined relative permittivity and the determined thickness of the pharmaceutical tablet by comparison to the reference data.

In addition, or alternatively, the controller comprises reference data correlating relative permittivity with hardness for a plurality of reference tablets of varying thickness and having the same chemical composition and other dimensions as the pharmaceutical tablet, and the controller is configured to determine the hardness of the pharmaceutical tablet using the determined relative permittivity and the determined thickness of the pharmaceutical tablet by comparison to the reference data.

In an embodiment of the device, in the first pair of conducting elements the conducting elements have flat surfaces of the same dimensions and/or in the second pair of conducting elements the conducting elements have flat surfaces of the same dimensions.

In a further embodiment the device comprises a conduit for the pharmaceutical tablet in which the one or more sensing sections are arranged, the conduit enabling the pharmaceutical tablet to pass through the device and the one or more sensing sections and the first capacitance measurement and the second capacitance measurement to be obtained.

The device may comprise shielding to shield the multiple capacitance sensors from fluctuations in external conditions, optionally wherein the shielding is electromagnetic shielding.

The device may also comprises one or more positioning elements to position the pharmaceutical tablet in the gap between the flat surfaces of the first pair of conducting elements and/or in the gap between the flat surfaces of the second pair of conducting elements.

In a further aspect the present invention provides a tableting unit for producing a plurality of pharmaceutical tablets, the tableting unit comprising a tablet manufacturing module (e.g. comprising a tablet press) and at least one device as described above positioned inline downstream of the tablet manufacturing module such that the plurality of pharmaceutical tablets pass through the device to allow the first capacitance and second capacitance measurements to be obtained. The tableting unit may comprise a plurality of the devices positioned inline downstream of the tablet manufacturing module, wherein the plurality of devices are arranged in series or in parallel to increase throughput and/or the number of pharmaceutical tablets for which the capacitance measurements can be obtained.

A further aspect of the invention is a method of determining a relative permittivity and a thickness of a pharmaceutical tablet using one or more capacitance sensors, wherein each of the one or more capacitance sensors comprises a pair of conducting elements, each conducting element of the pair having a flat surface, wherein the flat surfaces of the pair of conducting elements are arranged in parallel such that the capacitance measurements can be obtained from a gap therebetween, the method comprising:
(a) obtaining a first differential capacitance measurement using one of the one or more capacitance sensors by measuring the capacitance between the flat surfaces of the pair of conducting elements with and without the pharmaceutical tablet positioned in the gap, wherein when the capacitance is measured with the pharmaceutical tablet positioned in the gap the pharmaceutical tablet is in a first orientation in which the thickness of the pharmaceutical tablet is parallel to the flat surfaces of the pair of conducting elements;
(b) obtaining a second differential capacitance measurement using one of the one or more capacitance sensors by measuring the capacitance between the flat surfaces of the pair of conducting elements with and without the pharmaceutical tablet positioned in the gap, wherein when the capacitance is measured with the pharmaceutical tablet positioned in the gap the pharmaceutical tablet is in a second orientation in which the thickness of the pharmaceutical tablet is perpendicular to the flat surfaces of the pair of conducting elements; and
(c) determining the relative permittivity and thickness of the pharmaceutical tablet using the first and second differential capacitance measurements;
wherein (a) and (b) are performed in either order.

A still further asepct of the invention is a method of determining the solid fraction and/or hardness of a pharmaceutical tablet, the method comprising: determining the relative permittivity and thickness of the pharmaceutical tablet according to (a) to (c) of the above aspect; (d) determining the solid fraction of the pharmaceutical tablet using the determined relative permittivity and the determined thickness of the pharmaceutical tablet by comparison to reference data, the reference data correlating relative permittivity with solid fraction for a plurality of reference tablets of varying thickness and having the same chemical composition and other dimensions as the pharmaceutical tablet; and/or (e) determining the hardness of the pharmaceutical tablet using the determined relative permittivity and the determined thickness of the pharmaceutical tablet by comparison to reference data, the reference data correlating relative permittivity with hardness for a plurality of reference tablets of varying thickness and having the same chemical composition and other dimensions as the pharmaceutical tablet.

In particular, in the above methods one or more of (c) to (e) may be performed on a computer.

In one embodiment of the above methods, one capacitance sensor is used to obtain the first differential capacitance measurement and the second differential capacitance measurement, and the method comprises reorientating the pharmaceutical tablet between the first orientation and the second orientation, or vice versa, between the measurements.

In an alternative embodiment of the above methods, the one or more capacitance sensors are a first capacitance sensor for obtaining the first differential capacitance measurement and a second capacitance sensor for obtaining the second differential capacitance sensor, the first capacitance sensor comprising a first pair of conducting elements and the second capacitance sensor comprising a second pair of conducting elements.

In particular, the first capacitance sensor and the second capacitance sensor may be arranged in a device in one or more sensing sections.

For example, the first capacitance sensor and the second capacitance sensor may be arranged in the device in one sensing section in which the flat surfaces of the first pair of conducting elements are arranged perpendicularly to the flat surfaces of the second pair of conducting elements, and the method comprises positioning the pharmaceutical tablet in the first orientation with respect to the flat surfaces of the first pair of conducting elements which is also the second orientation with respect to the flat surfaces of the second pair of conducting elements.

In another example, the first capacitance sensor is arranged in a first sensing section and the second capacitance sensor is arranged in a second sensing sections, wherein the method comprises reorientating the pharmaceutical tablet between first orientation and the second orientation or between the second orientation and the first orientation as the pharmaceutical tablet is moved between the first and second sensing sections, or vice versa.

In a further example, the first capacitance sensor is arranged in a first sensing section and the second capacitance sensor is arranged in a second sensing section, wherein the flat surfaces of the first pair of conducting elements are arranged perpendicularly to the flat surfaces of the second pair of conducting elements, and the method comprises moving the pharmaceutical tablet between the first and second sensing sections, or between the second and first sensing sections.

In an embodiment of the methods the pair of conducting elements are a pair of parallel plates.

In a further embodiment of the methods the pair of conducting elements have flat surfaces of the same dimensions.

The methods may comprise obtaining the capacitance measurements while the pharmaceutical tablet is stationary, optionally while the pharmaceutical tablet is stationary in the one or more sensing sections.

Alternatively, the methods may comprise obtaining the capacitance measurements while the pharmaceutical tablet is moving, optionally wherein the pharmaceutical tablet is moving through the one or more sensing sections.

The methods may comprise using an Out-of-Phase technique to eliminate interference with the capacitance measurements.

The methods may be implemented inline in a tableting unit.

In a still further aspect, the present invention provides a pharmaceutical tableting process comprising producing pharmaceutical tablets using a tablet manufacturing module and determining the relative permittivity and a thickness of the pharmaceutical tablets according to the methods described above. In particular, the tablet manufacturing module may comprise a tablet press.

In an additional aspect the present invention provides for a use of the device described above to determine a relative permittivity and thickness of a pharmaceutical tablet. In particular, the use may be one in which the relative permittivity and thickness are determined according to the method described above.

In particular, in one aspect the present invention discloses an inline, fast and non-destructive quantitative measurement method and device, based on electrical tomography, for determination of solid fraction and thickness of tablets in a pharmaceutical tableting process. Tablet hardness can also be inferred in a non-destructive way. The device comprises (or consists of) multiple sensors in one or more sensing sections that can be used to obtain fast, non-invasive and non-destructive inline measurements that can be used to determine tablet thickness and solid fraction.

The present invention uses only electrical tomography to determine these quantities and does not require additional devices and technologies.

Regarding its application, when installed at the outlet of the tablet press, the device of the invention can assure the quality of the pharmaceutical tableting process throughout the batch, increasing sample representativity while reducing the need for destructive off-line experimentation, and promoting the implementation of Real Time Release due to the information it is able to generate. The device also has the potential to be a critical and widespread tool in the definition of control strategy for several applications (for example, in Continuous Manufacturing). The destructive and sporadic tablet analysis can be replaced by a non-destructive and highly frequent determination.

Multiple devices can be installed in, either in series or in parallel, for increased throughput and scale-up, to increase batch sample representativeness (i.e. sample analysis throughout the batch).

### FIGURES

To assist understanding of the present disclosure and to show how embodiments may be put into effect, reference is made by way of example to the accompanying drawings in which:
Figure 1 provides a representation of a device and dimensions for multiple measurements in single section according to an embodiment of the invention. The representation shows a device (4), a first capacitance sensor having a first pair of conducting elements (1a and 1b), a second capacitance sensor having a second pair of conducting elements (2a and 2b), and a pharmaceutical tablet (3).
Figure 2 provides a scheme for multiple capacitance measurements in a single section according to an embodiment of the invention.
Figure 3 provides a representation of a device with multiple capacitance measurements in two sensing areas according to an embodiment of the invention. The representation shows a device (4), a first capacitance sensor (1), having a first pair of conducting elements (1a and 1b), a second capacitance sensor (2) having a second pair of conducting elements (2a and 2b), and a pharmaceutical tablet (3) having a thickness (*dₜ*).
Figure 4 provides a scheme showing a capacitance sensor and tablet according to an embodiment of the invention.
Figure 5 provides a graph of calculated relative permittivity vs tablet density obtained in Example 1.
Figure 6 provides a graph of calculated relative permittivity vs tablet hardness obtained in Example 1.
Figure 7 provides a graph of calculated tablet thickness vs measured tablet thickness obtained in Example 1.
Figure 8 provides a representation of capacitance measurements obtained in Example 2 when a tablet is passing dynamically through a device described herein.

### DETAILED DESCRIPTION OF THE INVENTION

The description of the invention provided herein uses the terms "first" and "second" e.g. referring to "a first capacitance sensor", "a second capacitance sensor", "a first capacitance measurement", and "a second capacitance measurement". The use of the terms "first" and "second" herein is for identification purposes only and does not refer to an order of use or an order in which the described method steps are to be taken. In particular, the "first capacitance sensor" and "second capacitance sensor" in the device described herein may be for use in any order to obtain the capacitance measurements and may be arranged in series in the device in any order, and in the methods described herein the "first capacitance measurement" and the "second capacitance measurement" may be obtained in any order.

The present invention discloses a high-resolution electrical tomography-based device, capable of performing a non-destructive inline quantitative method for determination of relative permittivity and thickness of pharmaceutical tablets, from which solid fraction and hardness of tablets may be inferred, for example in a pharmaceutical tableting process.

In a first aspect the present invention provides a device for obtaining capacitance measurements for use in determining the relative permittivity and thickness of a pharmaceutical tablet, the device comprising multiple capacitance sensors in one or more sensing sections, the multiple capacitance sensors comprising:
(i) a first capacitance sensor for obtaining a first capacitance measurement, the first capacitance sensor comprising a first pair of conducting elements each conducting element having a flat surface, wherein the flat surfaces of the first pair of conducting elements are arranged in parallel such that capacitance measurements can be obtained from a gap therebetween, wherein the first capacitance measurement is to be obtained when the pharmaceutical tablet is positioned in the gap between the flat surfaces of the first pair of conducting elements in a first orientation with respect to the flat surfaces of the first pair of conducting elements;
(ii) a second capacitance sensor for obtaining a second capacitance measurement, the second capacitance sensor comprising a second pair of conducting elements each having a flat surface, wherein the flat surfaces of the second pair of conducting elements are arranged in parallel such that capacitance measurements can be obtained from a gap therebetween, wherein the second capacitance measurement is to be obtained when the pharmaceutical tablet is positioned in the gap between the flat surfaces of the second pair of conducting elements in a second orientation with respect to the flat surfaces of the second pair of conducting elements;

wherein the arrangement of the parallel flat surfaces of the first pair of conducting elements relative to the parallel flat surfaces of the second pair of conducting elements enables the first orientation to be perpendicular to the second orientation such that in one of the first and second orientations the thickness of the pharmaceutical tablet is parallel to the parallel flat surfaces of the pair of conducting elements and in the other of the first and second orientations the thickness of the pharmaceutical tablet is perpendicular to the parallel flat surfaces of the pair of conducting elements, and
wherein the device comprises a controller, wherein the controller is configured to determine the relative permittivity and thickness of the pharmaceutical tablet using the first and second capacitance measurements.

As described above, the capacitance sensors for use in the device comprise a pair of conducting elements, each having a flat surface, wherein the flat surfaces are arranged in parallel with a gap (or operation space) in between from which capacitance measurements can be obtained. The conducting elements may be of any 3D conformation having a flat surface, however, preferably the pair of conducting elements are two parallel plates.

The flat surfaces may be of any shape, e.g. rectangular, square, circular, or ringshaped.

The flat surfaces of the conducting elements have area (A), and are arranged in parallel separated by a distance (d), which defines the gap between the flat surfaces. Preferably the flat surface of a conducting element has the same dimensions as the flat surface of the other conducting element in the same pair.

Preferably the area (A) and distance (d) of the first pair of conducting elements are the same as the area (A) and distance (d) of the second pair of conducting elements.

The accuracy of the measurements obtained by the capacitance sensors can be increased or optimized by minimizing the free space around the pharmaceutical tablet when it is positioned in the gap in the first and/or second orientations.

To obtain capacitance measurements the conducting elements are connected to a power source in order to generate an electric field in the gap between the flat surfaces of the conducting elements and to obtain the capacitance measurements from the gap.

The first and second capacitance measurements are to be obtained using the device when the pharmaceutical tablet is in a first orientation with respect to the parallel flat surfaces of the first pair of conducting elements and when the pharmaceutical tablet is in a second orientation with respect to the parallel flat surfaces of the second pair of conducting elements. In particular, the device is configured to enable the first orientation to be perpendicular to the second orientation such that in one of the first and second orientations the thickness of the pharmaceutical tablet is parallel to the parallel flat surfaces of the pair of conducting elements of the capacitance sensor obtaining the measurement and in the other of the first and second orientations the thickness of the pharmaceutical tablet is perpendicular to the parallel flat surfaces of the pair of conducting elements of the capacitance sensor obtaining the measurement. In other words, in one of the first and second orientations the capacitance flow lines are perpendicular to the thickness of the pharmaceutical tablet and in the other of the first and second orientations the capacitance flow lines are parallel to the thickness of the tablet.

As used herein the term "perpendicular" refers to 90°± 5°, preferably 90° ± 2°, more preferably 90° ± 1°, most preferably 90° ± 0.5°, in order to obtain as accurate a determination of the relative permittivity and thickness of the pharmaceutical tablet as possible.

In particular, some variability around the 90° angle is permissible, and may be necessitated by the limitations of the sensor/device manufacture. However, it is considered that the most accurate determinations may be obtained with an angle as close as possible to 90°.

Similarly, the term "parallel" as used herein includes a variation of ± 5° from parallel, preferably ± 2°, more preferably ± 1°, most preferably ± 0.5° from parallel. Again, it is considered that accuracy is improved by keeping this variation as low as possible.

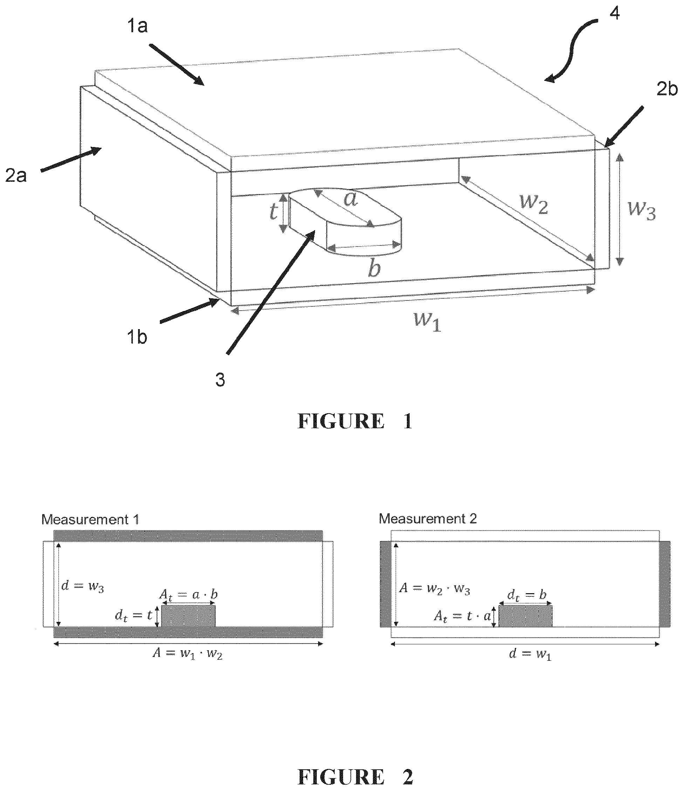

Examples of suitable configurations for the first and second capacitance sensors in the device to enable these capacitance measurements to be taken are shown in Figures 1 to 3.

In the method of the invention, as discussed below, the two capacitance measurements can be obtained by one capacitance sensor, with the orientation of the pharmaceutical tablet with respect to the flat surfaces of the conducting elements of the capacitance sensor being changed between the first orientation and the second orientation, or vice versa, between the different capacitance measurements. This can be done either by hand or using a machine.

However, the device described herein comprises two capacitance sensors, a first capacitance sensor for obtaining the first capacitance measurement and a second capacitance sensor for obtaining the second capacitance measurement.

The two capacitance sensors may be arranged in parallel in one sensing section, for example as shown in Figures 1 and 2. In this arrangement the device may be box-shaped. In particular, Figure 1 shows an example of a device with one sensing section in which the parallel flat surfaces of the first pair of conducting elements (1a and 1b) are arranged perpendicularly to the parallel flat surfaces of the second pair of conducting elements (2a and 2b). In this example, in the first orientation (in which measurement 1 of Figure 2 is taken) the thickness (t) of the pharmaceutical tablet (3) is perpendicular to the parallel flat surfaces of the first pair of conducting elements, while in the second orientation (in measurement 2 of Figure 2 is taken) the thickness (t) of the pharmaceutical tablet (3) is parallel to the parallel flat surfaces of the second pair of conducting elements.

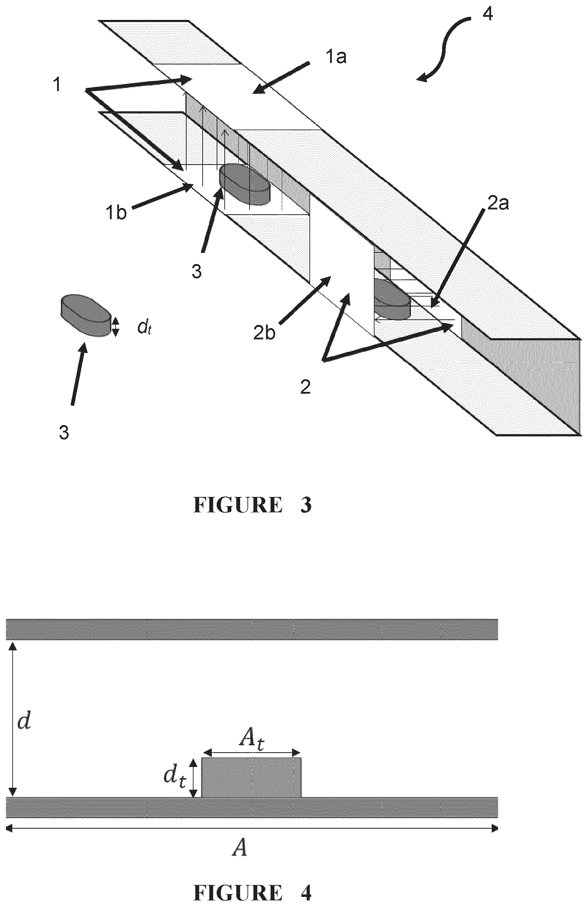

Alternatively, the two capacitance sensors may be arranged in series in two sensing sections. The sensing sections may be immediately adjacent or be two distinct sensing sections or areas. A schematic of a possible configuration using two distinct sensing areas is shown in Figure 3. In particular, Figure 3 shows an example of a device with two sensing sections in which the parallel flat surfaces of the first pair of conducting elements (1a and 1b) are arranged perpendicularly to the parallel flat surfaces of the second pair of conducting elements (2a and 2b). In this example, in the first orientation of the pharmaceutical tablet (as shown by the tablet indicated between the conducting elements 1a and 1b) the thickness (*dₜ*) of the pharmaceutical tablet (3) is perpendicular to the parallel flat surfaces of the first pair of conducting elements and the capacitance flow lines are parallel to the thickness(*dₜ*). In the second orientation of the pharmaceutical tablet (as shown by the tablet indicated between the conducting elements 2a and 2b) the thickness (*dₜ*) of the pharmaceutical tablet (3) is parallel to the parallel flat surfaces of the first pair of conducting elements and the capacitance flow lines are perpendicular to the thickness(*dₜ*).

The device may be configured to enable the first and second capacitance measurements to be perpendicular to each other as described above through the arrangement of the parallel flat surfaces of the second conducting elements with respect to the parallel flat surfaces of the first conducting elements. For example, as per the embodiment shown in Figures 1 and 3, the parallel flat surfaces of the first pair of conducting elements may be arranged perpendicularly to the parallel flat surfaces of the second pair of conducting elements, e.g. one capacitance sensor has its conducting elements on each side of an opening or conduit, while the other has it conducting elements on the top and bottom of the opening or conduit.

Alternatively, in an example that is not within the scope of claim 1, where the device comprises two sensing sections arranged in series in a first sensing section and a second sensing section, and the device may be configured to enable the first and second capacitance measurements to be perpendicular to each other by comprising a reorientation element to reorientate the pharmaceutical tablet between the first sensing section and the second sensing section, or vice versa, depending on which sensing section the pharmaceutical tablet enters first. (As noted above this may be in any order.) The reorientation element may comprise a funnel or other mechanical means.

The device may be configured to enable the capacitance measurements to be taken while the pharmaceutical tablet is stationary in the one or more sensing sections, or while the pharmaceutical tablet is moving through the one or more sensing sections. In particular, the device may comprise a conduit (e.g. a tube) to allow the pharmaceutical tablet to pass through the one or more sensing sections. The conduit may be horizontal or at an angle with respect to the ground. For example, the conduit (e.g. a tube) may be horizontal to ensure that the pharmaceutical tablet is stationary in the one or more sensing sections, or may be at an angle with respect to the ground to allow the pharmaceutical tablet to move through the one or more sensing sections under the influence of gravity. Preferably the conduit is at an angle to ensure that the pharmaceutical tablet moves quickly through the one or more sensing sections to ensure a high throughput. Alternative means may also be used to move the pharmaceutical tablet through the one or more sensing sections, e.g. where the conduit is horizontal. Such means include for example a conveyor or a pneumatic system.

When the pharmaceutical tablet is moving through the device, as discussed further below, multiple capacitance measurements may be taken while the pharmaceutical tablet passes (in the first orientation or in the second orientation) through the gap between the flat surfaces of the conductor elements.

The order of the first and second capacitance measurements is not relevant, in particular as both are used together to solve the system of equations set out below.

The device may comprise a connection part for connection to a controller. Further the device comprises a controller that is configured to determine the relative permittivity and thickness of the pharmaceutical tablet using the first and second capacitance measurements obtained using the device. For example, the device may comprise an integrated chip.

In particular, the controller may comprise reference data correlating relative permittivity with solid fraction for a plurality of reference tablets of varying thickness and having the same chemical composition and other dimensions as the pharmaceutical tablet. For example, where the pharmaceutical tablet has been produced by a tablet press the area of the upper surface of the pharmaceutical tablet is known as this is determined by the punches installed in the press, e.g. for a circular tablet the circular surface area of the tablet is known. The reference data may comprise sets of data points for pharmaceutical tablets having the same upper surface area and chemical composition as the pharmaceutical tablet, but a range of relative permittivities and tablet thicknesses, correlated to solid fraction and/or hardness. Such reference data sets may be established using existing off-line methods. (For example, the thickness of reference tablets may be determined using multi-tester instruments such as those currently used in commercial pharmaceutical tablet production processes, and the hardness may be determined using traditional destructive tests.)

The controller may also be configured to determine the solid fraction and/or hardness of the pharmaceutical tablet using the determined relative permittivity and the determined thickness of the pharmaceutical tablet by comparison to the reference data.

The device may be configured to use multiple channels that allow the use of an Out-of-Phase (OoP) technique, eliminating any interference such as a human proximity either nearby or in contact with the apparatus. Using this technique the measurements can be asynchronous. The device may include one or more environmental reference sensors to help compensate for environmental changes. The device may also include shielding, optionally electromagnetic shielding, to protect the capacitance sensors from fluctuations in external conditions. The shielding may be active or passive. An example of passive shielding is a layer made of metal, such as tin.

The device may also comprise a positioning element to position the pharmaceutical tablet in the gap between the flat surfaces of the first pair of conducting elements and/or in the gap between the flat surfaces of the second pair of conducting elements. The positioning element may be used to ensure that the tablets (unless round) are aligned with the conducting elements of the sensor. In particular, the positioning element may be a funnel or a chute, and may ensure that the pharmaceutical tablet is positioned a fixed distance from the flat surfaces of the conducting elements.

In alternative examples, the alignment of the tablet with the flat surfaces of the conducting elements can use a force, e.g. a centripetal or gravitational force.

The device may comprise positioning sensors to detect the position of the pharmaceutical tablet. In particular, if no alignment is ensured, more tomography measurement can be used to detect the position of the pharmaceutical tablet.

The positioning element may also be a reorientation element as described above. For example, the device may comprise a first positioning element to ensure that the pharmaceutical tablet (which is otherwise in the first orientation) is aligned (straight) with respect to the parallel flat surfaces of the conducting elements of the first capacitance sensor in the first sensing section, and a second positioning element which reorientates the pharmaceutical tablet from the first orientation into the second orientation before the second sensing section and ensures that the pharmaceutical tablet in the second orientation is aligned with respect to the parallel flat surfaces of the conducting element of the second capacitance sensor.

As is known in the art, it is preferable that the device is arranged such that the position of the pharmaceutical tablet is away from the edges of the conducting elements due to the fringe effect of the capacitance.

As described above, the device of the invention has particular utility inline in a pharmaceutical tableting process. Accordingly, in a further aspect the present invention provides a tableting unit for producing a plurality of pharmaceutical tablets, the tableting unit comprising a tablet manufacturing module and at least one device as described herein positioned inline downstream of the tablet manufacturing module such that the plurality of pharmaceutical tablets pass through the device to allow the first capacitance and second capacitance measurements to be obtained. The tableting unit may comprise a plurality of the devices described herein arranged in series or in parallel. In particular, this will increase throughput and/or the number of pharmaceutical tablets being assessed by the device. Preferably the tablet manufacturing module is a tablet press.

The tableting unit may further comprise a sorting means for sorting the pharmaceutical tablets based on the determined thickness, solid fraction and/or hardness.

The tableting unit may further comprise a feedback system for adjusting the production parameters of the tablet manufacturing module based on the determined thickness, solid fraction and/or hardness.

The pharmaceutical tablet can have any composition or shape. For example, the pharmaceutical tablet can be circular, rectangular, oblong or torpedo-shaped. In a preferred embodiment, the pharmaceutical tablet is circular.

It is preferred that the pharmaceutical tablet has been produced from a tablet press. In particular, as noted above, the pharmaceutical tablets from a tablet press have a known surface area (as determined by the shape of the punches installed in the press), and the method has particular utility in determining the thickness of such tablets.

The device described above can be used to independently perform the capacitance measurement in high-resolution of the pharmaceutical tablet (i.e. the target object) which is located between the conducting elements of the first and second capacitance sensors (either statically or dynamically). Thus, two types of signals are obtained which can then be correlated with different properties of the tablet - since the relative permittivity of the sample is the same, it is possible to determine both the thickness and solid fraction (hardness can also be inferred). This design is a critical part of this invention, as it allows to use a single technology (electrical tomography) to extract all the necessary information of the tablet without relying on other technologies or additional devices, resulting in a simpler and more compact apparatus.

In a further aspect the invention provides a method of determining a relative permittivity and a thickness of a pharmaceutical tablet using one or more capacitance sensors, wherein each of the one or more capacitance sensors comprises a pair of conducting elements, each conducting element of the pair having a flat surface, wherein the flat surfaces of the pair of conducting elements are arranged in parallel such that the capacitance measurements can be obtained from a gap therebetween, the method comprising:
(a) obtaining a first differential capacitance measurement using one of the one or more capacitance sensors by measuring the capacitance between the flat surfaces of the pair of conducting elements with and without the pharmaceutical tablet positioned in the gap, wherein when the capacitance is measured with the pharmaceutical tablet positioned in the gap the pharmaceutical tablet is in a first orientation in which the thickness of the pharmaceutical tablet is parallel to the flat surfaces of the pair of conducting elements;
(b) obtaining a second differential capacitance measurement using one of the one or more capacitance sensors by measuring the capacitance between the flat surfaces of the pair of conducting elements with and without the pharmaceutical tablet positioned in the gap, wherein when the capacitance is measured with the pharmaceutical tablet positioned in the gap the pharmaceutical tablet is in a second orientation in which the thickness of the pharmaceutical tablet is perpendicular to the flat surfaces of the pair of conducting elements; and
(c) determining the relative permittivity and thickness of the pharmaceutical tablet using the first and second differential capacitance measurements;
wherein (a) and (b) are performed in either order.

A further aspect is a method of determining the solid fraction and/or hardness of a pharmaceutical tablet, the method comprising: determining the relative permittivity and thickness of the pharmaceutical tablet according the method of the above paragraph; and (d) determining the solid fraction of the pharmaceutical tablet using the determined relative permittivity and the determined thickness of the pharmaceutical tablet by comparison to reference data, the reference data correlating relative permittivity with solid fraction for a plurality of reference tablets of varying thickness and having the same chemical composition and other dimensions as the pharmaceutical tablet; and/or (e) determining the hardness of the pharmaceutical tablet using the determined relative permittivity and the determined thickness of the pharmaceutical tablet by comparison to reference data, the reference data correlating relative permittivity with hardness for a plurality of reference tablets of varying thickness and having the same chemical composition and other dimensions as the pharmaceutical tablet.

In a preferred embodiment one or more of steps (c) to (e) recited above are performed on a computer or a controller.

The description of the features of the invention provided above in relation to the device also apply to the same features that are recited in the methods of the invention. However, also as noted above, in one embodiment of the method of the invention one capacitance sensor is used to obtain both the first differential capacitance measurement and the second differential capacitance measurement, wherein the pharmaceutical tablet is moved between the first orientation and the second orientation (or vice versa) between the measurements.

The method may comprise one or more steps of positioning the pharmaceutical tablet in the gap between the flat surfaces of the pair of conducting elements. In particular, the positioning step can be used to align the pharmaceutical tablet with the flat surfaces of the conducting elements of the capacitor(s) so that it is straight. Where the pharmaceutical tablet is round such positioning to align the tablet is not required.

In one example of the invention the device described above is used to perform the method.

The method may comprise using an Out-of-Phase technique as described above to eliminate any interference in the capacitance measurements from the external environment of the capacitance sensors. Suitable techniques are known in the art from the use of capacitive sensing to determine height of liquids in tanks or containers.

In the method, obtaining the capacitance measurements can be performed while the pharmaceutical tablet stationary in the one or more sensing sections, or while the pharmaceutical tablet is moving through the one or more sensing sections. Preferably the method is performed with the tablet moving through the one or more sensing sections, since this provides a higher throughput. High throughput is necessary when the method is used inline in a pharmaceutical tableting process.

In particular, in one embodiment the method is implemented inline in a tableting unit.

Accordingly, in a further aspect, the invention provides a pharmaceutical tableting process comprising producing pharmaceutical tablets using a tablet manufacturing module and determining the relative permittivity and a thickness of the pharmaceutical tablets according to the methods described herein. Preferably the tablet manufacturing module comprises a tablet press.

In the methods described herein, once the solid fraction and/or hardness has been determined, this information can be further used to sort the tablets and/or to adjust the parameters of production of the tablet manufacturing module, e.g. the tablet press, via a feedback loop. Therefore, the methods described herein may comprise a step of sorting the pharmaceutical tablets according to their determined thickness, solid fraction and/or hardness, and/or a step of adjusting the production parameters of the tablet manufacturing module which produced the pharmaceutical tablets so as to alter the thickness, solid fraction and/or hardness of subsequently produced tablets.

This method is not derived in an obvious way from the prior art as it is the specific measurement conditions, i.e. the orientation of the pharmaceutical tablet with respect to the flat surfaces of the conducting elements during the first and second capacitance measurements, that allow the determination of the thickness using a tomography sensor.

The determination of relative permittivity and thickness is explained further as below:

### Determination of relative permittivity

The capacitance measurement is useful in this application as their sensors respond to the mass, density and volume of material present between the plates. Therefore, by difference, it is possible to obtain the capacitance of the material located between the parallel plates.

The capacitance of parallel plates having the same dimensions is described by the following equation: $C = \frac{{ϵ}_{0} {ϵ}_{r} A}{d}$

Where *ε*₀ is the permittivity of the vacuum, *εᵣ* is the relative permittivity of the medium, *A* the area of the plates and d the distance between the plates.

Since the sample only occupies a section of the gap between the sensor plates, it is necessary to apply the equations for capacitors in series and in parallel to have a representative equation for the system. Using a differential measurement between the sensor, with and without the sample, it is possible to obtain the equation below: $ΔC = {\left[\frac{{d}_{t}}{{ϵ}_{0} {ϵ}_{r} {A}_{t}}+\frac{d - {d}_{t}}{{ϵ}_{0} {A}_{t}}\right]}^{- 1} - \frac{{ϵ}_{0} {A}_{t}}{d}$

Where *Aₜ* is the sample area, *dₜ* the sample thickness and *εᵣ* the sample relative permittivity of the sample.

Rearranging equation 2, it is possible to derive the relative permittivity, which is directly related to the solid fraction and hardness of tablets, using a simple calibration. ${ϵ}_{r} = 1 + {\left[\frac{{d}_{t}}{d}\frac{{ϵ}_{0} ⋅ {A}_{t}}{Δ Cd}+\frac{{d}_{t}}{d}-1\right]}^{- 1}$

This equation is part of the current state of the art and is also mentioned in US20200217811A1.

### Thickness determination

The design and properties of the sensor plates are well known and part of the construction of the apparatus -gap distance (d) and vacuum permittivity (*ε*₀). The tablet area (*Aₜ*) is also known and solely depends on the punches installed in the press and used to manufacture the tablet.

The thickness of the pharmaceutical tablet is required to estimate the solid fraction. This value is not constant for all tablets being produced (e.g. from a tablet press) as there are non-negligible variations between the tablets. Therefore, the determination of the thickness is an important part of this invention.

This invention proposes to use another (a second or further) capacitance measurement to be able to estimate the thickness. This allows for a single technology to be used to extract all the necessary information from the tablet without relying on other technologies, resulting in a simpler and more compact apparatus.

The equation to determine the thickness of a generic tablet can be deduced. Thus, ·considering thickness *t* and width *b* (second measurement perpendicular to the tablet width) and length *a*: ${\left[\frac{1}{{w}_{3}}\frac{{ϵ}_{0} a ⋅ b ⋅ {Φ}_{shape}}{Δ {C}_{1} {w}_{3}}+\frac{1}{{w}_{3}}-\frac{b *}{{w}_{1}}\frac{{ϵ}_{0} a}{Δ {C}_{2} {w}_{1}}\right]}^{- 1} \frac{b *}{{w}_{1}} = t$

Where Δ*C*₁ and Δ*C*₂ are the first and second capacitance measurements, *w*₁, *w*₂ and *w*₃ are the dimensions of the sensor, Φ*ₛₕₐₚₑ* is the shape factor of the tablet*.
* It can be required to add a shape factor Φ*ₛₕₐₚₑ* to compute the area of the tablet. This shape factor will be the same for each tablet shape and only dependent on the equipment that has been used to produce the pharmaceutical tablet (for example, Φ*_{snage}* = *π*/4 for round tablets). Despite b*(width of the equivalent rectangular tablet) will not have the exact same value as b (tablet width of tablet), it will be directly proportional to the width, provided that a small correction to the roundness of the tablet is incorporated.

A similar equation can be obtained for all tablet shapes and sizes.

### Determination of solid fraction and hardness

The relative permittivity and thickness determined for the pharmaceutical tablet are then used to determine the solid fraction and/or hardness of the pharmaceutical tablet.

For each type of pharmaceutical tablet (having a particular composition and particular dimensions and shape) a reference data set can be created relating to a plurality of reference tablets. This data set correlates relative permittivity to a solid fraction and/or hardness value for the plurality of reference tablets having varying thicknesses (each reference tablet having a different thickness). Accordingly, the determined relative permittivity and thickness for a pharmaceutical tablet can be used to determine the solid fraction and/or hardness value through correlation with the reference data set.

The following are intended as examples only and do not limit the present disclosure.

### EXAMPLES

### Example 1

A prototype of the invention was used, the prototype having two capacitance sensors arranged in one sensing section (similar to Figure 1), with the parallel plates of the first capacitance sensor being arranged perpendicularly to the parallel plates of the second capacitance sensor. The prototype was tested using placebo pharmaceutical tablets. The tested tablets were round, with a mass range between 121g and 132g and a thickness range between 3.4mm and 4.1mm.

As described before, two independent capacitance measurements were made for each tablet using the first and second capacitance sensors and the equations previously described were applied to compute their thickness and relative permittivity.

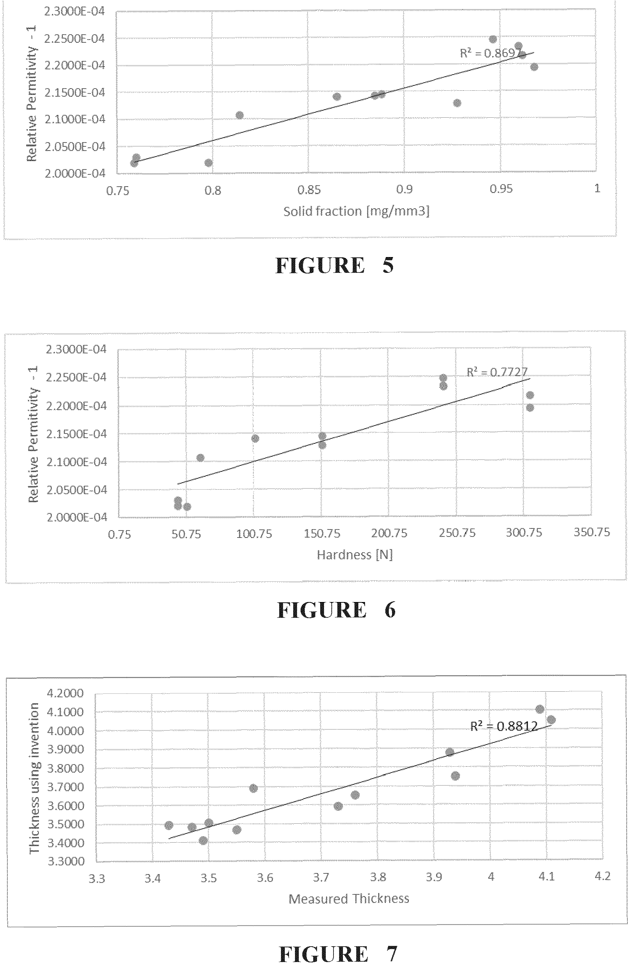

The data presented (Figures 5, 6 and 7) shows a good relation between the predicted and measured properties, which would not be achieved with a single capacitance measurement. This demonstrates the functionality of the invention as both tablet solid fraction and hardness are well correlated with the relative permittivity.

### Example 2

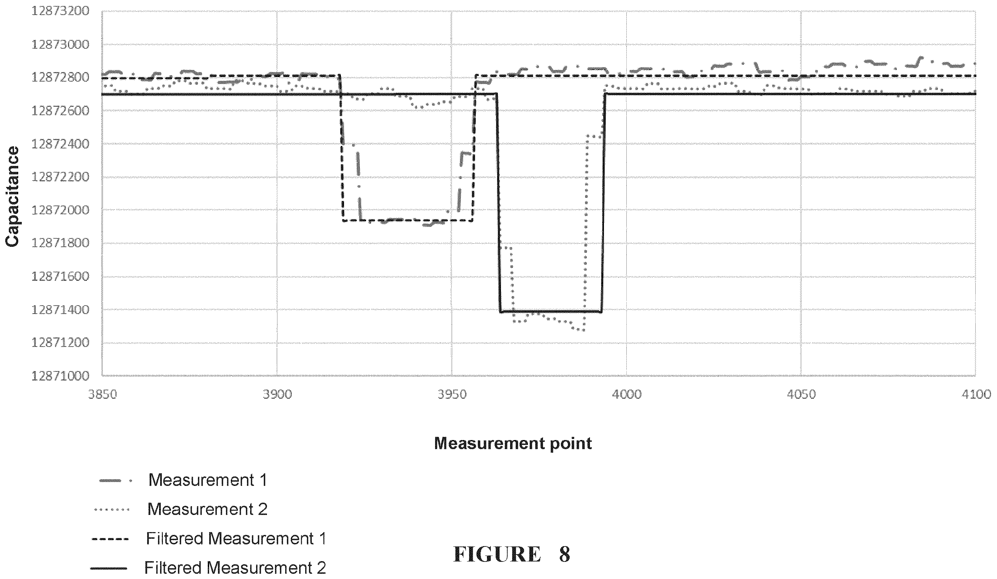

In another example, a device with two capacitance sensors arranged in two sensing areas in a tube (similar to Figure 3) was used, with the parallel plates of the second capacitance sensor in the second sensing area being arranged perpendicularly to the parallel plates of the first capacitance sensor in the first sensing area. The device was installed with an angle of approximately 45º to allow the tablets to pass through the device (along the tube) under the influence of gravity. The same tablets as in Example 1 were used. Capacitance measurements from the two capacitance sensors were obtained. Figure 8 shows an example of the capacitance measurement obtained with one tablet. It is observed that a good signal to noise ratio and a high frequency of analysis (3ms per measurement point) were achieved, which, based on the size of the sensors used in the example, allows roughly 30 stable measurement points per capacitance sensor as the tablet moves through the tube. In addition, the signal isolation of both sensors is also observed, which confirms the feasibility of the use of the device and method described herein inline in a pharmaceutical tableting process to achieve fast and high-resolution determination of the thickness and solid fraction of pharmaceutical tablets produced by the tablet press.

### REFERENCES

Beck, M, and RA Williams. 1995. Process Tomography: Principles, Techniques and Applications. Edited by Butterworth-Heinemann.
Beck, M, and R Williams. 1996. "Process Tomography: A European Innovation and Its Applications." Measurement Science and Technology 7: 215.
Crowe, CT. 2005. Multiphase Flow Handbook. Edited by CT Crowe, E Michaelides, and JD Schwarzkopf. Boca Raton: CRC Press - Taylor & Francis Group.
Dickin, F, R Waterfall, and R Williams. 1992. "Tomographic Imaging of Industrial Process Equipment: Techniques and Applications." Circuits, Devices and Systems, IEE Proceedings G 139 (I): 72-82.
Dyakowski, T, and AJ Jaworski. 2003. "Non-Invasive Process Imaging - Principles and Applications of Industrial Process Tomography." Chemical Engineering & Technology 26 (6): 697-706
Müller, J. et al. 2012 "Prediction of dissolution time and coating thickness of sustained release formulations using Raman spectroscopy and terahertz pulsed imaging", Eur J. Pharm and Biopharm 80: 690-697
Peeters et al 2016, "Assessment and prediction of tablet properties using transmission and backscattering Raman spectroscopy and transmission NIR spectroscopy", Asian J. Pharm Sci. 11: 547-558
Rimpilainen V., 2012, "Electrical tomography imaging in pharmaceutical processes", Publications of the University of Eastern Finland, Dissertations in Forestry and Natural Sciences, No 68
Bolton G.T., Primrose K.M., 2005, "An Overview of Electrical Tomographic Measurements in Pharmaceutical and Related Application Areas", AAPS PharmSciTech 2005; 6 (2) Article 21
Ehrhardt N., Montagne M., Berthiaux H., Dalloz-Dubrujeaud B., Gatumel C., 2005, Chemical Engineering and Processing: Process Intensification Volume 44, Issue 2, February 2005, Pages 303-313

## Claims

1. A device (4) for obtaining capacitance measurements for use in determining the relative permittivity and thickness (*d*ₜ, *t*) of a pharmaceutical tablet (3), the device comprising multiple capacitance sensors in one or more sensing sections, the multiple capacitance sensors comprising:
(i) a first capacitance sensor (1) for obtaining a first capacitance measurement, the first capacitance sensor comprising a first pair of conducting elements (1a, 1b) each conducting element having a flat surface, wherein the flat surfaces of the first pair of conducting elements are arranged in parallel such that capacitance measurements can be obtained from a gap therebetween, wherein the first capacitance measurement is to be obtained when the pharmaceutical tablet is positioned in the gap between the flat surfaces of the first pair of conducting elements in a first orientation with respect to the flat surfaces of the first pair of conducting elements;
(ii) a second capacitance sensor (2) for obtaining a second capacitance measurement, the second capacitance sensor comprising a second pair of conducting elements (2a, 2b) each having a flat surface, wherein the flat surfaces of the second pair of conducting elements are arranged in parallel such that capacitance measurements can be obtained from a gap therebetween, wherein the second capacitance measurement is to be obtained when the pharmaceutical tablet is positioned in the gap between the flat surfaces of the second pair of conducting elements in a second orientation with respect to the flat surfaces of the second pair of conducting elements;
wherein the arrangement of the parallel flat surfaces of the first pair of conducting elements relative to the parallel flat surfaces of the second pair of conducting elements enables the first orientation to be perpendicular to the second orientation such that in one of the first and second orientations the thickness of the pharmaceutical tablet is parallel to the parallel flat surfaces of the pair of conducting elements and in the other of the first and second orientations the thickness of the pharmaceutical tablet is perpendicular to the parallel flat surfaces of the pair of conducting elements, and
wherein the device comprises a controller, wherein the controller is configured to determine the relative permittivity and thickness of the pharmaceutical tablet using the first and second capacitance measurements.

2. The device (4) of claim 1, wherein the device comprises:
(i) one sensing section in which the flat surfaces of the first pair of conducting elements (1a, 1b) are arranged perpendicularly to the flat surfaces of the second pair of conducting elements (2a, 2b); or
(ii) two sensing sections arranged in series, wherein the first pair of conducting elements (1a, 1b) are arranged in the first sensing section and the second pair of conducting elements are arranged in the second sensing section, wherein the second pair of conducting elements (2a, 2b) are arranged perpendicularly to the first pair of conducting elements.

3. The device (4) of claim 1 or claim 2, wherein the first pair of conducting elements (1a, 1b) are a first pair of parallel plates and the second pair of conducting elements (2a, 2b) are a second pair of parallel plates.

4. The device (4) of any preceding claim, wherein the controller comprises:
(i) reference data correlating relative permittivity with solid fraction for a plurality of reference tablets of varying thickness and having the same chemical composition and other dimensions as the pharmaceutical tablet (3), and the controller is configured to determine the solid fraction of the pharmaceutical tablet using the determined relative permittivity and the determined thickness (dt, *t*) of the pharmaceutical tablet by comparison to the reference data; and/or
(ii) reference data correlating relative permittivity with hardness for a plurality of reference tablets of varying thickness and having the same chemical composition and other dimensions as the pharmaceutical tablet (3), and the controller is configured to determine the hardness of the pharmaceutical tablet using the determined relative permittivity and the determined thickness (dt, *t*) of the pharmaceutical tablet by comparison to the reference data.

5. The device (4) of any preceding claim, wherein in the first pair of conducting elements (1a, 1b) the conducting elements have flat surfaces of the same dimensions and/or in the second pair of conducting elements (2a, 2b) the conducting elements have flat surfaces of the same dimensions.

6. The device (4) according to any preceding claim, wherein the device comprises one or more of:
(i) a conduit for the pharmaceutical tablet in which the one or more sensing sections are arranged, the conduit enabling the pharmaceutical tablet (3) to pass through the device and the one or more sensing sections and the first capacitance measurement and the second capacitance measurement to be obtained;
(ii) shielding to shield the multiple capacitance sensors from fluctuations in external conditions, optionally wherein the shielding is electromagnetic shielding; and
(iii) one or more positioning elements to position the pharmaceutical tablet in the gap between the flat surfaces of the first pair of conducting elements (1a, 1b) and/or in the gap between the flat surfaces of the second pair of conducting elements (2a, 2b).

7. A tableting unit for producing a plurality of pharmaceutical tablets, the tableting unit comprising a tablet manufacturing module and one or a plurality of devices (4) of any one of claims 1 to 6 positioned inline downstream of the tablet manufacturing module such that the plurality of pharmaceutical tablets (3) pass through the one or the plurality of device(s) to allow the first capacitance and second capacitance measurements to be obtained, wherein when the tableting unit comprises the plurality of devices, the plurality of devices are arranged in series or in parallel to increase throughput and/or the number of pharmaceutical tablets (3) for which the capacitance measurements can be obtained,
optionally wherein the tablet manufacturing module comprises a tablet press.

8. A method of determining a relative permittivity and a thickness (*d*ₜ, *t*) of a pharmaceutical tablet (3) using one or more capacitance sensors (1, 2), wherein each of the one or more capacitance sensors comprises a pair of conducting elements (1a, 1b, 2a, 2b), each conducting element of the pair having a flat surface, wherein the flat surfaces of the pair of conducting elements are arranged in parallel such that the capacitance measurements can be obtained from a gap therebetween, the method comprising:
(a) obtaining a first differential capacitance measurement using one of the one or more capacitance sensors by measuring the capacitance between the flat surfaces of the pair of conducting elements with and without the pharmaceutical tablet positioned in the gap, wherein when the capacitance is measured with the pharmaceutical tablet positioned in the gap the pharmaceutical tablet is in a first orientation in which the thickness of the pharmaceutical tablet is parallel to the flat surfaces of the pair of conducting elements;
(b) obtaining a second differential capacitance measurement using one of the one or more capacitance sensors by measuring the capacitance between the flat surfaces of the pair of conducting elements with and without the pharmaceutical tablet positioned in the gap, wherein when the capacitance is measured with the pharmaceutical tablet positioned in the gap the pharmaceutical tablet is in a second orientation in which the thickness of the pharmaceutical tablet is perpendicular to the flat surfaces of the pair of conducting elements; and
(c) determining the relative permittivity and thickness of the pharmaceutical tablet using the first and second differential capacitance measurements;
wherein (a) and (b) are performed in either order.

9. A method of determining the solid fraction and/or hardness of a pharmaceutical tablet (3), the method comprising: determining the relative permittivity and thickness (*d*ₜ, *t*) of the pharmaceutical tablet according claim 8 (a) to (c); (d) determining the solid fraction of the pharmaceutical tablet using the determined relative permittivity and the determined thickness of the pharmaceutical tablet by comparison to reference data, the reference data correlating relative permittivity with solid fraction for a plurality of reference tablets of varying thickness and having the same chemical composition and other dimensions as the pharmaceutical tablet; and/or (e) determining the hardness of the pharmaceutical tablet using the determined relative permittivity and the determined thickness of the pharmaceutical tablet by comparison to reference data, the reference data correlating relative permittivity with hardness for a plurality of reference tablets of varying thickness and having the same chemical composition and other dimensions as the pharmaceutical tablet,
optionally wherein one or more of (c) to (e) are performed on a computer.

10. The method of claim 8 or claim 9, wherein:
(i) one capacitance sensor (1, 2) is used to obtain the first differential capacitance measurement and the second differential capacitance measurement, and the method comprises reorientating the pharmaceutical tablet (3) between the first orientation and the second orientation, or vice versa, between the measurements; or
(ii) the one or more capacitance sensors are a first capacitance sensor (1) for obtaining the first differential capacitance measurement and a second capacitance sensor (2) for obtaining the second differential capacitance measurement, the first capacitance sensor comprising a first pair of conducting elements (1a, 1b) and the second capacitance sensor comprising a second pair of conducting elements (2a, 2b).

11. The method of claim 10 part (ii), wherein the first capacitance sensor (1) and the second capacitance sensor (2) are arranged in a device (4) in one or more sensing sections, optionally wherein:
(i) the first capacitance sensor (1) and the second capacitance sensor (2) are arranged in the device (4) in one sensing section in which the flat surfaces of the first pair of conducting elements (1a, 1b) are arranged perpendicularly to the flat surfaces of the second pair of conducting elements (2a, 2b), and the method comprises positioning the pharmaceutical tablet (3) in the first orientation with respect to the flat surfaces of the first pair of conducting elements which is also the second orientation with respect to the flat surfaces of the second pair of conducting elements;
(ii) the first capacitance sensor (1) is arranged in a first sensing section and the second capacitance sensor is arranged in a second sensing sections, wherein the method comprises reorientating the pharmaceutical tablet (3) between first orientation and the second orientation or between the second orientation and the first orientation as the pharmaceutical tablet is moved between the first and second sensing sections, or vice versa;or
(iii) the first capacitance sensor (1) is arranged in a first sensing section and the second capacitance sensor (2) is arranged in a second sensing section, wherein the flat surfaces of the first pair of conducting elements (1a, 1b) are arranged perpendicularly to the flat surfaces of the second pair of conducting elements (2a, 2b), and the method comprises moving the pharmaceutical tablet (3) between the first and second sensing sections, or between the second and first sensing sections.

12. The method of any of claims 8 to 11, wherein:
(i) the pair of conducting elements (1a, 1b, 2a, 2b) are a pair of parallel plates and/or have flat surfaces of the same dimensions;
(ii) the method comprises using an Out-of-Phase technique to eliminate interference with the capacitance measurements; and/or
(iii) the method is implemented inline in a tableting unit.

13. The method of any of claims 8 to 12, comprising:
(i) obtaining the capacitance measurements while the pharmaceutical tablet (3) is stationary; or
(ii) obtaining the capacitance measurements while the pharmaceutical tablet (3) is moving.

14. A pharmaceutical tableting process comprising producing pharmaceutical tablets (3) using a tablet manufacturing module and determining the relative permittivity and a thickness (*d*ₜ, *t*) of the pharmaceutical tablets according to the method of any one of claims 8 to 13,
optionally wherein the tablet manufacturing module comprises a tablet press.

15. Use of a device (4) according to any of claims 1 to 6 to determine a relative permittivity and thickness (*d*ₜ, *t*) of a pharmaceutical tablet (3),
optionally wherein the relative permittivity and thickness (dt, *t*) are determined according to the method of any of claims 8 to 13.

## Patentansprüche

1. Vorrichtung (4) zum Beziehen von Kapazitätsmessungen zur Verwendung beim Bestimmen der relativen Dielektrizitätskonstante und der Dicke (*dₜ*, *t*) einer pharmazeutischen Tablette (3), wobei die Vorrichtung mehrere Kapazitätssensoren in einem oder mehreren Erfassungsabschnitten umfasst, wobei die mehreren Kapazitätssensoren umfassen:
(i) einen ersten Kapazitätssensor (1) zum Beziehen einer ersten Kapazitätsmessung, wobei der erste Kapazitätssensor ein erstes Paar von leitenden Elementen (1a, 1b) umfasst, wobei jedes leitende Element eine ebene Fläche aufweist, wobei die ebenen Flächen des ersten Paars von leitenden Elementen parallel eingerichtet sind, so dass Kapazitätsmessungen aus einem Spalt dazwischen bezogen werden können, wobei die erste Kapazitätsmessung zu beziehen ist, wenn die pharmazeutische Tablette in dem Spalt zwischen den ebenen Flächen des ersten Paars von leitenden Elementen in einer ersten Ausrichtung in Bezug auf die ebenen Flächen des ersten Paars von leitenden Elementen positioniert ist;
(ii) einen zweiten Kapazitätssensor (2) zum Beziehen einer zweiten Kapazitätsmessung, wobei der zweite Kapazitätssensor ein zweites Paar von leitenden Elementen (2a, 2b) umfasst, wobei jedes leitende Element eine ebene Fläche aufweist, wobei die ebenen Flächen des zweiten Paars von leitenden Elementen parallel eingerichtet sind, so dass Kapazitätsmessungen aus einem Spalt dazwischen bezogen werden können, wobei die zweite Kapazitätsmessung zu beziehen ist, wenn die pharmazeutische Tablette in dem Spalt zwischen den ebenen Flächen des zweiten Paars von leitenden Elementen in einer zweiten Ausrichtung in Bezug auf die ebenen Flächen des zweiten Paars von leitenden Elementen positioniert ist;
wobei die Einrichtung der parallelen ebenen Flächen des ersten Paars von leitenden Elementen in Bezug auf die parallelen ebenen Flächen des zweiten Paars von leitenden Elementen ermöglicht, dass die erste Ausrichtung senkrecht zu der zweiten Ausrichtung ist, so dass die Dicke der pharmazeutischen Tablette in einer von der ersten und der zweiten Ausrichtung parallel zu den parallelen ebenen Flächen des Paars von leitenden Elementen ist und die Dicke der pharmazeutischen Tablette in der anderen von der ersten und der zweiten Ausrichtung senkrecht zu den parallelen ebenen Flächen des Paars von leitenden Elementen ist, und
wobei die Vorrichtung eine Steuereinheit umfasst, wobei die Steuereinheit dazu konfiguriert ist, die relative Dielektrizitätskonstante und die Dicke der pharmazeutischen Tablette unter Verwendung der ersten und der zweiten Kapazitätsmessung zu bestimmen.

2. Vorrichtung (4) nach Anspruch 1, wobei der Vorrichtung umfasst:
(i) einen Erfassungsabschnitt, in dem die ebenen Flächen des ersten Paars von leitenden Elementen (1a, 1b) senkrecht zu den ebenen Flächen des zweiten Paars von leitenden Elementen (2a, 2b) eingerichtet sind; oder
(ii) zwei in Reihe eingerichtete Erfassungsabschnitte, wobei das erste Paar von leitenden Elementen (1a, 1b) in dem ersten Erfassungsabschnitt eingerichtet ist und das zweite Paar von leitenden Elementen in dem zweiten Erfassungsabschnitt eingerichtet ist, wobei das zweite Paar von leitenden Elementen (2a, 2b) senkrecht zu dem ersten Paar von leitenden Elementen eingerichtet ist.

3. Vorrichtung (4) nach Anspruch 1 oder Anspruch 2, wobei das erste Paar von leitenden Elementen (1a, 1b) ein erstes Paar von parallelen Platten ist und das zweite Paar von leitenden Elementen (2a, 2b) ein zweites Paar von parallelen Platten ist.

4. Vorrichtung (4) nach einem vorhergehenden Anspruch, wobei die Steuereinheit umfasst:
(i) Referenzdaten, die eine relative Dielektrizitätskonstante mit einem Feststoffanteil für eine Vielzahl von Referenztabletten von variierender Dicke und mit derselben chemischen Zusammensetzung und anderen Abmessungen als die pharmazeutische Tablette (3) korrelieren, und wobei die Steuereinheit dazu konfiguriert ist, den Feststoffanteil der pharmazeutischen Tablette unter Verwendung der bestimmten relativen Dielektrizitätskonstante und der bestimmten Dicke (*dₜ*, *t*) der pharmazeutischen Tablette durch Vergleich mit den Referenzdaten zu bestimmen; und/oder
(ii) Referenzdaten, die eine relative Dielektrizitätskonstante mit einer Härte für eine Vielzahl von Referenztabletten von variierender Dicke und mit derselben chemischen Zusammensetzung und anderen Abmessungen als die pharmazeutische Tablette (3) korrelieren, und wobei die Steuereinheit dazu konfiguriert ist, die Härte der pharmazeutischen Tablette unter Verwendung der bestimmten relativen Dielektrizitätskonstante und der bestimmten Dicke (*dₜ*, *t*) der pharmazeutischen Tablette durch Vergleich mit den Referenzdaten zu bestimmen.

5. Vorrichtung (4) nach einem vorhergehenden Anspruch, wobei die leitenden Elemente in dem ersten Paar von leitenden Elementen (1a, 1b) ebene Flächen von denselben Abmessungen aufweisen und/oder die leitenden Elemente in dem zweiten Paar von leitenden Elementen (2a, 2b) ebene Flächen von denselben Abmessungen aufweisen.

6. Vorrichtung (4) nach einem vorhergehenden Anspruch, wobei die Vorrichtung eine bzw. eines oder mehrere der folgenden umfasst:
(i) eine Leitung für die pharmazeutische Tablette, in der der eine oder die mehreren Erfassungsabschnitte eingerichtet sind, wobei die Leitung ermöglicht, dass die pharmazeutische Tablette (3) durch die Vorrichtung und den einen oder die mehreren Erfassungsabschnitte hindurchtritt und die erste Kapazitätsmessung und die zweite Kapazitätsmessung bezogen werden;
(ii) eine Abschirmung, um die mehreren Kapazitätssensoren von Schwankungen von äußeren Bedingungen abzuschirmen, optional wobei die Abschirmung eine elektromagnetische Abschirmung ist; und
(iii) ein oder mehrere Positionierelemente, um die pharmazeutische Tablette in dem Spalt zwischen den ebenen Flächen des ersten Paars von leitenden Elementen (1a, 1b) und/oder in dem Spalt zwischen den ebenen Flächen des zweiten Paars von leitenden Elementen (2a, 2b) zu positionieren.

7. Tablettiereinheit zur Produktion einer Vielzahl von pharmazeutischen Tabletten, wobei die Tablettiereinheit ein Tablettenherstellungsmodul und eine oder einer Vielzahl von Vorrichtungen (4) nach einem der Ansprüche 1 bis 6 umfasst, die inline dem Tablettenherstellungsmodul nachgelagert positioniert ist, so dass die Vielzahl von pharmazeutischen Tabletten (3) durch die eine oder die Vielzahl von Vorrichtungen hindurchtritt, um zu ermöglichen, dass die erste Kapazitätsmessung und die zweite Kapazitätsmessung bezogen werden, wobei, wenn die Tablettiereinheit die Vielzahl von Vorrichtungen umfasst, die Vielzahl von Vorrichtungen in Reihe oder parallel eingerichtet ist, um einen Durchsatz und/oder die Anzahl von pharmazeutischen Tabletten (3), für die die Kapazitätsmessungen bezogen werden können, zu erhöhen,
optional wobei das Tablettenherstellungsmodul eine Tablettenpresse umfasst.

8. Verfahren zur Bestimmung einer relativen Dielektrizitätskonstante und einer Dicke (*dₜ*, *t*) einer pharmazeutischen Tablette (3) unter Verwendung eines oder mehrerer Kapazitätssensoren (1, 2), wobei jeder von dem einen oder den mehreren Kapazitätssensoren ein Paar von leitenden Elementen (1a, 1b, 2a, 2b) umfasst, wobei jedes leitende Element des Paars eine ebene Fläche aufweist, wobei die ebenen Flächen des Paars von leitenden Elementen parallel eingerichtet sind, so dass die Kapazitätsmessungen aus einem Spalt dazwischen bezogen werden können, wobei das Verfahren umfasst:
(a) Beziehen einer ersten Differenzialkapazitätsmessung unter Verwendung eines von dem einen oder den mehreren Kapazitätssensoren durch Messen der Kapazität zwischen den ebenen Flächen des Paars von leitenden Elementen mit der und ohne die in dem Spalt positionierte pharmazeutische Tablette, wobei, wenn die Kapazität mit der in dem Spalt positionierten pharmazeutischen Tablette gemessen wird, die pharmazeutische Tablette in einer ersten Ausrichtung ist, in der die Dicke der pharmazeutischen Tablette parallel zu den ebenen Flächen des Paars von leitenden Elementen ist;
(b) Beziehen einer zweiten Differenzialkapazitätsmessung unter Verwendung eines von dem einen oder den mehreren Kapazitätssensoren durch Messen der Kapazität zwischen den ebenen Flächen des Paars von leitenden Elementen mit der und ohne die in dem Spalt positionierte pharmazeutische Tablette, wobei, wenn die Kapazität mit der in dem Spalt positionierten pharmazeutischen Tablette gemessen wird, die pharmazeutische Tablette in einer zweiten Ausrichtung ist, in der die Dicke der pharmazeutischen Tablette senkrecht zu den ebenen Flächen des Paars von leitenden Elementen ist; und
(c) Bestimmen der relativen Dielektrizitätskonstante und der Dicke der pharmazeutischen Tablette unter Verwendung der ersten und der zweiten Differenzialkapazitätsmessung;
wobei (a) und (b) in einer der beiden Reihenfolgen durchgeführt werden.

9. Verfahren zur Bestimmung des Feststoffanteils und/oder der Härte einer pharmazeutischen Tablette (3), wobei das Verfahren umfasst: Bestimmen der relativen Dielektrizitätskonstante und der Dicke (*dₜ*, *t*) der pharmazeutischen Tablette nach Anspruch 8, (a) bis (c); (d) Bestimmen des Feststoffanteils der pharmazeutischen Tablette unter Verwendung der bestimmten relativen Dielektrizitätskonstante und der bestimmten Dicke der pharmazeutischen Tablette durch Vergleich mit Referenzdaten, wobei die Referenzdaten eine relative Dielektrizitätskonstante mit einem Feststoffanteil für eine Vielzahl von Referenztabletten von variierender Dicke und mit derselben chemischen Zusammensetzung und anderen Abmessungen als die pharmazeutische Tablette korrelieren; und/oder (e) Bestimmen der Härte der pharmazeutischen Tablette unter Verwendung der bestimmten relativen Dielektrizitätskonstante und der bestimmten Dicke der pharmazeutischen Tablette durch Vergleich mit Referenzdaten, wobei die Referenzdaten eine relative Dielektrizitätskonstante mit einer Härte für eine Vielzahl von Referenztabletten von variierender Dicke und mit derselben chemischen Zusammensetzung und anderen Abmessungen als die pharmazeutische Tablette korrelieren,
optional wobei ein oder mehrere von (c) bis (e) auf einem Computer durchgeführt werden.

10. Verfahren nach Anspruch 8 oder Anspruch 9, wobei:
(i) ein Kapazitätssensor (1, 2), dazu verwendet wird, die erste Differenzialkapazitätsmessung und die zweite Differenzialkapazitätsmessung zu beziehen, und das Verfahren ein Neuausrichten der pharmazeutischen Tablette (3) zwischen der ersten Ausrichtung und der zweiten Ausrichtung oder umgekehrt zwischen den Messungen umfasst; oder
(ii) der eine oder die mehreren Kapazitätssensoren ein erster Kapazitätssensor (1) zum Beziehen der ersten Differenzialkapazitätsmessung und ein zweiter Kapazitätssensor (2) zum Beziehen der zweiten Differenzialkapazitätsmessung sind, wobei der erste Kapazitätssensor ein erstes Paar von leitenden Elementen (1a, 1b) umfasst und der zweite Kapazitätssensor ein zweites Paar von leitenden Elementen (2a, 2b) umfasst.

11. Verfahren nach Anspruch 10, Teil (ii), wobei der erste Kapazitätssensor (1) und der zweite Kapazitätssensor (2) in einer Vorrichtung (4) in einem oder mehreren Erfassungsabschnitten eingerichtet sind, optional wobei:
(i) der erste Kapazitätssensor (1) und der zweite Kapazitätssensor (2) in der Vorrichtung (4) in einem Erfassungsabschnitt eingerichtet sind, in dem die ebenen Flächen des ersten Paars von leitenden Elementen (1a, 1b) senkrecht zu den ebenen Flächen des zweiten Paars von leitenden Elementen (2a, 2b) eingerichtet sind, und das Verfahren ein Positionieren der pharmazeutischen Tablette (3) in der ersten Ausrichtung in Bezug auf die ebenen Flächen des ersten Paars von leitenden Elementen umfasst, die ebenfalls die zweite Ausrichtung in Bezug auf die ebenen Flächen des zweiten Paars von leitenden Elementen ist;
(ii) der erste Kapazitätssensor (1) in einem ersten Erfassungsabschnitt eingerichtet ist und der zweite Kapazitätssensor in einem zweiten Erfassungsabschnitt eingerichtet ist, wobei das Verfahren ein Neuausrichten der pharmazeutischen Tablette (3) zwischen einer ersten Ausrichtung und der zweiten Ausrichtung oder zwischen der zweiten Ausrichtung und der ersten Ausrichtung, während die pharmazeutische Tablette zwischen dem ersten und dem zweiten Erfassungsabschnitt bewegt wird, oder umgekehrt umfasst; oder
(iii) der erste Kapazitätssensor (1) in einem ersten Erfassungsabschnitt eingerichtet ist und der zweite Kapazitätssensor (2) in einem zweiten Erfassungsabschnitt eingerichtet ist, wobei die ebenen Flächen des ersten Paars von leitenden Elementen (1a, 1b) senkrecht zu den ebenen Flächen des zweiten Paars von leitenden Elementen (2a, 2b) eingerichtet sind und das Verfahren ein Bewegen der pharmazeutischen Tablette (3) zwischen dem ersten und dem zweiten Erfassungsabschnitt oder zwischen dem zweiten und dem ersten Erfassungsabschnitt umfasst.

12. Verfahren nach einem der Ansprüche 8 bis 11, wobei:
(i) das Paar von leitenden Elementen (1a, 1b, 2a, 2b) ein Paar von parallelen Platten ist und/oder ebene Flächen von denselben Abmessungen aufweist;
(ii) das Verfahren ein Verwenden einer phasenverschobenen Technik umfasst, um eine Interferenz mit den Kapazitätsmessungen zu eliminieren; und/oder
(iii) das Verfahren inline in einer Tablettiereinheit implementiert wird.

13. Verfahren nach einem der Ansprüche 8 bis 12, umfassend:
(i) Beziehen der Kapazitätsmessungen, während die pharmazeutische Tablette (3) ortsgebunden ist; oder
(ii) Beziehen der Kapazitätsmessungen, während sich die pharmazeutische Tablette (3) bewegt.

14. Pharmazeutischer Tablettierprozess, umfassend ein Produzieren von pharmazeutischen Tabletten (3) unter Verwendung eines Tablettenherstellungsmoduls und ein Bestimmen der relativen Dielektrizitätskonstante und einer Dicke (*dₜ*, *t*) der pharmazeutischen Tabletten gemäß dem Verfahren nach einem der Ansprüche 8 bis 13,
optional wobei das Tablettenherstellungsmodul eine Tablettenpresse umfasst.

15. Verwendung einer Vorrichtung (4) nach einem der Ansprüche 1 bis 6, um eine relative Dielektrizitätskonstante und eine Dicke (*dₜ*, *t*) einer pharmazeutischen Tablette (3) zu bestimmen,
optional wobei die relative Dielektrizitätskonstante und die Dicke (*dₜ*, *t*) gemäß dem Verfahren nach einem der Ansprüche 8 bis 13 bestimmt werden.

## Revendications

1. Un dispositif (4) pour obtenir des mesures de capacité pour les utiliser dans la détermination de la permittivité relative et de l'épaisseur (*dₜ, t)* d'un comprimé pharmaceutique (3), le dispositif comprenant des capteurs de capacité multiples dans une ou plusieurs sections de détection, les capteurs de capacité multiples comprenant :
(i) un premier capteur de capacité (1) pour obtenir une première mesure de capacité, le premier capteur de capacité comprenant une première paire d'éléments conducteurs (1a, 1b) chaque élément conducteur ayant une surface plate, dans lequel les surfaces plates de la première paire d'éléments conducteurs sont agencées en parallèle de telle sorte que des mesures de capacité peuvent être obtenues à partir d'un espace entre les surfaces plates, dans lequel la première mesure de capacité doit être obtenue quand le comprimé pharmaceutique est positionné dans l'espace entre les surfaces plates de la première paire d'éléments conducteurs dans une première orientation par rapport aux surfaces plates de la première paire d'éléments conducteurs ;
(ii) un deuxième capteur de capacité (2) pour obtenir une deuxième mesure de capacité, le deuxième capteur de capacité comprenant une deuxième paire d'éléments conducteurs (2a, 2b) chacun ayant une surface plate, dans lequel les surfaces plates de la deuxième paire d'éléments conducteurs sont agencées en parallèle de telle sorte que des mesures de capacité peuvent être obtenues à partir d'un espace entre les surfaces plates, dans lequel la deuxième mesure de capacité doit être obtenue quand le comprimé pharmaceutique est positionné dans l'espace entre les surfaces plates de la deuxième paire d'éléments conducteurs dans une deuxième orientation par rapport aux surfaces plates de la deuxième paire d'éléments conducteurs ;
dans lequel l'agencement des surfaces plates parallèles de la première paire d'éléments conducteurs par rapport aux surfaces plates parallèles de la deuxième paire d'éléments conducteurs permettent à la première orientation d'être perpendiculaire à la deuxième orientation de telle sorte que, dans une orientation, soit la première orientation, soit la deuxième orientation, l'épaisseur du comprimé pharmaceutique est parallèle aux surfaces plates parallèles de la paire d'éléments conducteurs et, dans l'autre orientation, soit la première orientation, soit la deuxième orientation, l'épaisseur du comprimé pharmaceutique est perpendiculaire aux surfaces plates parallèles de la paire d'éléments conducteurs, et
dans lequel le dispositif comprend une unité de commande, dans lequel l'unité de commande est configurée pour déterminer la permittivité relative et l'épaisseur du comprimé pharmaceutique en utilisant les première et deuxième mesures de capacité.

2. Le dispositif (4) selon la revendication 1, dans lequel le dispositif comprend :
(i) une section de détection dans laquelle les surfaces plates de la première paire d'éléments conducteurs (1a, 1b) sont agencées perpendiculairement aux surfaces plates de la deuxième paire d'éléments conducteurs (2a, 2b) ; ou
(ii) deux sections de détection agencées en série, dans lesquelles la première paire d'éléments conducteurs (1a, 1b) sont agencés dans la première section de détection et la deuxième paire d'éléments conducteurs sont agencés dans la deuxième section de détection, dans lesquelles la deuxième paire d'éléments conducteurs (2a, 2b) sont agencés perpendiculairement à la première paire d'éléments conducteurs.

3. Le dispositif (4) selon la revendication 1 ou la revendication 2, dans lequel la première paire d'éléments conducteurs (1a, 1b) sont une première paire de plaques parallèles et la deuxième paire d'éléments conducteurs (2a, 2b) sont une deuxième paire de plaques parallèles.

4. Le dispositif (4) selon l'une quelconque des revendications précédentes, dans lequel l'unité de commande comprend :
(i) des données de référence corrélant la permittivité relative avec la fraction solide pour une pluralité de comprimés de référence de différentes épaisseurs et ayant la même composition chimique et d'autres dimensions que le comprimé pharmaceutique (3), et l'unité de commande est configurée pour déterminer la fraction solide du comprimé pharmaceutique en utilisant la permittivité relative déterminée et l'épaisseur déterminée *(dₜ, t)* du comprimé pharmaceutique par comparaison avec les données de référence ; et/ou
(ii) des données de référence corrélant la permittivité relative avec la dureté pour une pluralité de comprimés de référence de différentes épaisseurs et ayant la même composition chimique et d'autres dimensions que le comprimé pharmaceutique (3), et l'unité de commande est configurée pour déterminer la dureté du comprimé pharmaceutique en utilisant la permittivité relative déterminée et l'épaisseur déterminée *(dₜ, t)* du comprimé pharmaceutique par comparaison avec les données de référence.

5. Le dispositif (4) selon l'une quelconque des revendications précédentes, dans lequel, dans la première paire d'éléments conducteurs (1a, 1b), les éléments conducteurs ont des surfaces plates des mêmes dimensions et/ou dans la deuxième paire d'éléments conducteurs (2a, 2b) les éléments conducteurs ont des surfaces plates des mêmes dimensions.

6. Le dispositif (4) selon l'une quelconque des revendications précédentes, dans lequel le dispositif comprend un ou plusieurs éléments parmi :
(i) un conduit pour le comprimé pharmaceutique dans lequel la ou les sections de détection sont agencées, le conduit permettant au comprimé pharmaceutique (3) de passer à travers le dispositif et la ou les sections de détection et la première mesure de capacité et la deuxième mesure de capacité à obtenir ;
(ii) une protection pour protéger les capteurs de capacité multiples contre les fluctuations des conditions externes, en option dans lequel la protection est une protection électromagnétique ; et
(iii) un ou plusieurs éléments de positionnement pour positionner le comprimé pharmaceutique dans l'espace entre les surfaces plates de la première paire d'éléments conducteurs (1a, 1b) et/ou dans l'espace entre les surfaces plates de la deuxième paire d'éléments conducteurs (2a, 2b).

7. Une unité de formation de comprimés pour produire une pluralité de comprimés pharmaceutiques, l'unité de formation de comprimés comprenant un module de fabrication de comprimés et un ou plusieurs dispositifs (4) selon l'une quelconque des revendications 1 à 6, positionnés en ligne en aval du module de fabrication de comprimés de telle sorte que la pluralité de comprimés pharmaceutiques (3) passent à travers le ou les dispositif(s) pour permettre d'obtenir les première et deuxième mesures de capacité, dans lequel, quand l'unité de formation de comprimés comprend plusieurs dispositifs, les dispositifs sont agencés en série ou en parallèle pour augmenter le débit et/ou le nombre de comprimés pharmaceutiques (3) pour lesquels les mesures de capacité peuvent être obtenues,
en option, dans lequel le module de fabrication de comprimés comprend une presse à comprimés.

8. Un procédé de détermination d'une permittivité relative et d'une épaisseur (*dt, t)* d'un comprimé pharmaceutique (3) en utilisant un ou plusieurs capteurs de capacité (1, 2), dans lequel le ou les capteurs de capacité comprennent chacun une paire d'éléments conducteurs (1a, 1b, 2a, 2b), chaque élément conducteur de la paire ayant une surface plate, dans lequel les surfaces plates de la paire d'éléments conducteurs sont agencées en parallèle de telle sorte que les mesures de capacité peuvent être obtenues à partir d'un espace entre les surfaces plates, le procédé comprenant :
(a) l'obtention d'une première mesure de capacité différentielle en utilisant un des capteurs de capacité en mesurant la capacité entre les surfaces plates de la paire d'éléments conducteurs avec et sans le comprimé pharmaceutique positionné dans l'espace, dans lequel, quand la capacité est mesurée avec le comprimé pharmaceutique positionné dans l'espace, le comprimé pharmaceutique est dans une première orientation, dans laquelle l'épaisseur du comprimé pharmaceutique est parallèle aux surfaces plates de la paire d'éléments conducteurs ;
(b) l'obtention d'une deuxième mesure de capacité différentielle en utilisant un des capteurs de capacité en mesurant la capacité entre les surfaces plates de la paire d'éléments conducteurs avec et sans le comprimé pharmaceutique positionné dans l'espace, dans lequel, quand la capacité est mesurée avec le comprimé pharmaceutique positionné dans l'espace, le comprimé pharmaceutique est dans une deuxième orientation dans laquelle l'épaisseur du comprimé pharmaceutique est perpendiculaire aux surfaces plates de la paire d'éléments conducteurs ; et
(c) la détermination de la permittivité relative et de l'épaisseur du comprimé pharmaceutique en utilisant les première et deuxième mesures de capacité différentielles ;
dans lequel (a) et (b) sont effectués dans un ordre quelconque.

9. Un procédé de détermination de la fraction solide et/ou de la dureté d'un comprimé pharmaceutique (3), le procédé comprenant : la détermination de la permittivité relative et de l'épaisseur (*dₜ, t)* du comprimé pharmaceutique selon la revendication 8 (a) à (c) ; (d) la détermination de la fraction solide du comprimé pharmaceutique en utilisant la permittivité relative déterminée et l'épaisseur déterminée du comprimé pharmaceutique par comparaison avec des données de référence, les données de référence corrélant la permittivité relative avec la fraction solide pour une pluralité de comprimés de référence de différentes épaisseurs et ayant la même composition chimique et d'autres dimensions que le comprimé pharmaceutique ; et/ou (e) la détermination de la dureté du comprimé pharmaceutique en utilisant la permittivité relative déterminée et l'épaisseur déterminée du comprimé pharmaceutique par comparaison avec des données de référence, les données de référence corrélant la permittivité relative avec la dureté pour une pluralité de comprimés de référence de différentes épaisseurs et ayant les mêmes compositions chimiques et d'autres dimensions que le comprimé pharmaceutique,
en option dans lequel un ou plusieurs points de (c) à (e) sont effectués sur un ordinateur.

10. Le procédé selon la revendication 8 ou la revendication 9, dans lequel :
(i) un capteur de capacité (1, 2) est utilisé pour obtenir la première mesure de capacité différentielle et la deuxième mesure de capacité différentielle, et le procédé comprend la réorientation du comprimé pharmaceutique (3) entre la première orientation et la deuxième orientation, ou vice versa, entre les mesures ; ou
(ii) le ou les capteurs de capacité sont un premier capteur de capacité (1) pour obtenir la première mesure de capacité différentielle et un deuxième capteur de capacité (2) pour obtenir la deuxième mesure de capacité différentielle, le premier capteur de capacité comprenant une première paire d'éléments conducteurs (1a, 1b) et le deuxième capteur de capacité comprenant une deuxième paire d'éléments conducteurs (2a, 2b).

11. Le procédé selon la revendication 10 partie (ii), dans lequel le premier capteur de capacité (1) et le deuxième capteur de capacité (2) sont agencés dans un dispositif (4) dans une ou plusieurs sections de détection, en option dans lequel :
(i) le premier capteur de capacité (1) et le deuxième capteur de capacité (2) sont agencés dans le dispositif (4) dans une section de détection dans laquelle les surfaces plates de la première paire d'éléments conducteurs (1a, 1b) sont agencées perpendiculairement aux surfaces plates de la deuxième paire d'éléments conducteurs (2a, 2b), et le procédé comprend le positionnement du comprimé pharmaceutique (3) dans la première orientation par rapport aux surfaces plates de la première paire d'éléments conducteurs qui est aussi la deuxième orientation par rapport aux surfaces plates de la deuxième paire d'éléments conducteurs ;
(ii) le premier capteur de capacité (1) est agencé dans une première section de détection et le deuxième capteur de capacité est agencé dans une deuxième section de détection, dans lequel le procédé comprend la réorientation du comprimé pharmaceutique (3) entre la première orientation et la deuxième orientation ou entre la deuxième orientation et la première orientation lorsque le comprimé pharmaceutique est déplacé entre les première et deuxième sections de détection, ou vice versa ; ou
(iii) le premier capteur de capacité (1) est agencé dans une première section de détection et le deuxième capteur de capacité (2) est agencé dans une deuxième section de détection, dans lequel les surfaces plates de la première paire d'éléments conducteurs (1a, 1b) sont agencées perpendiculairement aux surfaces plates de la deuxième paire d'éléments conducteurs (2a, 2b), et le procédé comprend le déplacement du comprimé pharmaceutique (3) entre les première et deuxième sections de détection, ou entre les deuxième et première sections de détection.

12. Le procédé selon l'une quelconque des revendications 8 à 11, dans lequel :
(i) la paire d'éléments conducteurs (1a, 1b, 2a, 2b) sont une paire de plaques parallèles et/ou ont des surfaces plates des mêmes dimensions ;
(ii) le procédé comprend l'utilisation d'une technique de déphasage pour éliminer les interférences avec les mesures de capacité ; et/ou
(iii) le procédé est mis en œuvre en ligne dans une unité de formation de comprimés.

13. Le procédé selon l'une quelconque des revendications 8 à 12, comprenant :
(i) l'obtention des mesures de capacité pendant que le comprimé pharmaceutique (3) est fixe ; ou
(ii) l'obtention des mesures de capacité pendant que le comprimé pharmaceutique (3) se déplace.

14. Un processus de fabrication de comprimés pharmaceutiques comprenant la production de comprimés pharmaceutiques (3) en utilisant un module de fabrication de comprimés et la détermination de la permittivité relative et d'une épaisseur (*dₜ, t)* des comprimés pharmaceutiques conformément au procédé selon l'une quelconque des revendications 8 à 13,
en option dans lequel le module de fabrication de comprimés comprend une presse à comprimés.

15. Utilisation d'un dispositif (4) selon l'une quelconque des revendications 1 à 6 pour déterminer une permittivité relative et une épaisseur (*dₜ, t)* d'un comprimé pharmaceutique (3),
en option dans lequel la permittivité relative et l'épaisseur *(dₜ, t)* sont déterminées conformément au procédé selon l'une quelconque des revendications 8 à 13.
